Europäisches Patentamt

European Patent Office

Office européen des brevets

(19)

(11) **EP 0 750 667 B1**

(12) **EUROPÄISCHE PATENTSCHRIFT**

(45) Veröffentlichungstag und Bekanntmachung des Hinweises auf die Patenterteilung:
**23.12.1998  Patentblatt 1998/52**

(21) Anmeldenummer: **95913095.6**

(22) Anmeldetag: **13.03.1995**

(51) Int Cl.⁶: **C12N 15/15**, C12N 9/72,
C07K 14/81, C12N 1/19,
C12N 1/21, C12N 5/10

(86) Internationale Anmeldenummer:
**PCT/EP95/00926**

(87) Internationale Veröffentlichungsnummer:
**WO 95/25168 (21.09.1995 Gazette 1995/40)**

(54) **VERWENDUNG VON REKOMBINANTEM INHIBITOR AUS ERYTHRINA CAFFRA ZUR REINIGUNG VON SERINPROTEASEN**

USE OF A RECOMBINANT INHIBITOR FROM ERYTHRINA CAFFRA FOR PURIFYING SERINE PROTEASES

UTILISATION D'UN INHIBITEUR RECOMBINE ISSU D'ERYTHRINA CAFFRA POUR PURIFIER DES SERINE PROTEASES

(84) Benannte Vertragsstaaten:
**AT BE CH DE DK ES FR GB GR IE IT LI LU NL PT SE**

(30) Priorität: **16.03.1994  DE 4408939**
**08.07.1994  DE 4424171**

(43) Veröffentlichungstag der Anmeldung:
**02.01.1997  Patentblatt 1997/01**

(73) Patentinhaber: **BOEHRINGER MANNHEIM GMBH**
**68305 Mannheim (DE)**

(72) Erfinder:
• **KOHNERT, Ulrich**
**82392 Habach (DE)**
• **STERN, Anne**
**82377 Penzberg (DE)**
• **FISCHER, Stephan**
**82398 Polling (DE)**

(56) Entgegenhaltungen:
**EP-A- 0 218 479**

• **BIOCHIMICA BIOPHYSICA ACTA, Bd. 1217, Nr. 1, 1994 Seite 16-22 A. TEIXEIRA ET AL 'Synthesis and expression of a gene coding for Erythrina trypsin inhibitor (ETI)' in der Anmeldung erwähnt**
• **CHEMICAL ABSTRACTS, vol. 119, no. 11, 13.September 1993 Columbus, Ohio, US; abstract no. 111939, Y. KOUZUMA 'Isolation and primary structure of proteinase inhibitors from Erythrina variegata (Linn.) var. Orientalis seeds' Seite 397; & BIOSCI. BIOTECHNOL. BIOCHEM., Bd. 56, Nr. 11, 1992 Seiten 1819-1824,**
• **BIOCHEMISTRY, Bd. 22, 1983 Seiten 1621-1630, J. PORATH AND B. OLIN 'Immobilized metal ion affinity adsorption and immobolized metal ion affinity chromatography of biomaterials.' in der Anmeldung erwähnt**

EP 0 750 667 B1

**Beschreibung**

Die Erfindung betrifft ein verbessertes Verfahren zur Reinigung von Serinproteasen unter Verwendung von rekombinantem Inhibitor DE-3 aus Erythrina caffra. Immobilisierte Trypsin-Inhibitoren aus Erythrina (ETI) sind effektive Reagenzien für die affinitätschromatographische Reinigung von Serinproteasen, insbesondere von Plasminogenaktivatoren (C. Heussen, J. Biol. Chem. 259 (1984) 11635 - 11638), $\beta$-Trypsin, $\alpha$-Chymotrypsin und Thrombin (S. Onesti et al., J. Mol. Recogn. 5 (1992) 105 - 114). Diese Trypsin-Inhibitoren sind seit langem bekannt (C. Heussen, Haemostasis 11(1982) P47 (Supplement); F.J. Joubert, Phytochemistry 21 (1982) 1213- 1217; F.J. Joubert, Int. J. Biochem. 14 (1982) 187 - 193).

Der Inhibitor DE-3 aus E. caffra ist besonders bevorzugt zur affinitätschromatographischen Reinigung von Plasminogenaktivatoren geeignet (F.J. Joubert in Thrombosis and Haemostasis 57 (1987) 356 - 360). In dieser Publikation ist auch die komplette Aminosäuresequenz dieses Inhibitors beschrieben. DE-3 kann aus dem Samen von E. caffra isoliert und gereinigt werden (F.J. Joubert, Int. J. Biochem. 14 (1982) 187 - 193).

Von Teixeira et al., Biochimica et Biophysica Acta 1217 (1994) 16-22 wird ein rekombinanter ETI beschrieben, dessen spezifische inhibitorische Aktivität für Gewebsplasminogenaktivator bei $1,7 \times 10^9$ U/mmol liegt. Die spezifische inhibitorische Aktivität von natürlichem ETI liegt dagegen bei $1,94 \times 10^9$ U/mmol. Analoges gilt für die inhibitorische Aktivität gegenüber Trypsin ($2,63 \times 10^{12}/3,21 \times 10^{12}$). Damit ist die spezifische inhibitorische Aktivität von nach Teixeira hergestelltem rekombinantem ETI für Trypsin und Gewebsplasminogenaktivator um 20% geringer als die Aktivität von natürlichem ETI.

Rekombinanter ETI wird nach Teixeira durch Expression gewonnen und aufgereinigt durch eine Ammonsulfatfällung (80%ige Sättigung), Dialyse gegen Wasser und eine Cyanbromidspaltung, wobei die N-terminale Sequenz einschließlich des Methionins abgespalten wird. Anschließend wird eine Affinitätschromatographiesäule (Sephadex G50) chromatographiert.

Aufgabe der Erfindung ist die Verbesserung der Effektivität von Verfahren zur Reinigung von Serinproteasen unter Verwendung von ETI.

Gegenstand der Erfindung ist ein Verfahren zur Reinigung von Serinproteasen aus einem Proteingemisch durch Bindung der Serinprotease ein immobilisiertes Polypeptid, welches die Aktivität eines Inhibitors DE-3 aus Erythrina caffra besitzt, Entfernen der ungebundenen Anteile aus dem Proteingemisch, Ablösen der Serinprotease vom Inhibitor, Trennung des immobilisierten Inhibitors von der löslichen Serinprotease und Isolierung der Serinprotease, welches dadurch gekennzeichnet ist, daß als Polypeptid ein Polypeptid verwendet wird, das das Produkt einer prokaryontischen oder eukaryontischen Expression einer exogenen Nukleinsäure (vorzugsweise DNA) ist und chromatographisch über einen Anionenaustauscher, Kationenaustauscher oder eine Nickelchelatsäure gereinigt ist.

Überraschenderweise wurde gefunden, daß ein rekombinantes, erfindungsgemäß hergestelltes Polypeptid, welches die Aktivität eines Inhibitors DE-3 aus Erythrina caffra besitzt, im Gegensatz zum aus natürlichen Quellen isolierten Inhibitor DE-3 aus E.caffra eine deutlich erhöhte spezifische Affinität gegenüber Serinproteasen besitzt.

Unter "Aktivität" eines Inhibitors DE-3 aus E.caffra ist im wesentlichen seine spezifische inhibitorische Aktivität für Serinproteasen, insbesondere für Gewebsplasminogenaktivatoren zu verstehen. Die spezifische inhibitorische Aktivität des Inhibitors liegt dabei bei 1,07 U/mg oder höher gegenüber Trypsin. Die Inhibition erfolgt durch Bindung zwischen Inhibitor und Serinprotease.

Das erfindungsgemäße Verfahren ist besonders vorteilhaft zur Reinigung von Plasminogenaktivatoren, wie Gewebs-Plasminogen-Aktivatoren (t-PA) und Derivaten (z.B. Mutationen und Deletionen) davon, geeignet. t-PA und Derivate sind beispielsweise in der EP-B 0 093 619, USP 5,223,256, WO 90/09437 und T.J.R. Harris, Protein Engineering 1(1987) 449 - 458 beschrieben.

Die Herstellung des rekombinanten Inhibitors kann nach den dem Fachmann geläufigen Methoden erfolgen.

Dazu wird zunächst eine Nukleinsäure (vorzugsweise DNA) hergestellt, welche in der Lage ist, ein Protein zu produzieren, welches die Aktivität des Inhibitors DE-3 besitzt. Die DNA wird in einen Vektor kloniert, der in eine Wirtszelle transferiert werden kann und dort replizierbar ist. Ein derartiger Vektor enthält zusätzlich zur Inhibitorsequenz Operator-Elemente, die zur Expression der DNA erforderlich sind. Dieser Vektor, der die Inhibitor-DNA und die Operator-Elemente enthält, wird in einen Vektor transferiert, der in der Lage ist, die DNA des Inhibitors zu exprimieren. Die Wirtszelle wird unter Bedingungen kultiviert, welche die Expression des Inhibitors erlauben. Der Inhibitor wird aus diesen Zellen gewonnen. Dabei wird durch geeignete Maßnahmen sichergestellt, daß der Aktivator eine aktive tertiäre Struktur einnehmen kann, in der er Inhibitoreigenschaften zeigt.

Dabei ist es nicht erforderlich, daß der Inhibitor die exakte Aminosäuresequenz entsprechend SEQ ID NO:2 besitzt. Ebenso geeignet sind Inhibitoren, welche im wesentlichen die gleiche Sequenz besitzen und Polypeptide mit der Aktivität (Bindefähigkeit an Serinproteasen, insbesondere an t-PA) eines Inhibitors DE-3 aus Erythrina caffra sind. Es hat sich gezeigt, daß eine Übereinstimmung der Aminosäuresequenz von 80%, bevorzugt von 90%, vorteilhaft ist. Die Aminosäuresequenz SEQ ID NO:2 wird jedoch bevorzugt verwendet, wobei im Falle der Expression in prokaryontischen Wirtszellen, nicht jedoch nach eukaryontischer Expression, ein N-terminales Methionin enthalten ist.

Ein weiterer Gegenstand der Erfindung ist eine Nukleinsäure, welche im wesentlichen identisch ist mit den Nukleotiden 9 bis 527 von SEQ ID NO:1 und für ein Polypeptid mit der Aktivität eines Inhibitors DE-3 aus Erythrina caffra codiert, oder eine Nukleinsäure, die im Rahmen der Degeneration des genetischen Codes für das gleiche Polypeptid codiert. Bevorzugt ist eine DNA, insbesondere eine DNA der Sequenz 9 bis 527 der SEQ ID NO:1. Zur Expression in eukaryotischen oder prokaryontischen Wirtszellen enthält die Nukleinsäure am 5'-Ende die dem Fachmann geläufigen eukaryotischen oder prokaryontischen Transkriptions- und Translationssignale.

Die Nukleinsäure-Sequenz des Inhibitors ist vorzugsweise identisch mit den Nukleotiden 9 bis 527 der SEQ ID NO:1. Allerdings können zur Erleichterung der Herstellung der Vektoren oder Optimierung der Expression Modifikationen angebracht sein. Derartige Modifikationen sind beispielsweise:

- Veränderung der Nukleinsäure, um verschiedene Erkennungssequenzen von Restriktionsenzymen zur Erleichterung der Schritte der Ligation, Klonierung und Mutagenese einzuführen
- Veränderung der Nukleinsäure zum Einbau von bevorzugten Codons für die Wirtszelle
- Ergänzung der Nukleinsäure um zusätzliche Operator-Elemente, um die Expression in der Wirtszelle zu optimieren.

Die Expression des Inhibitors erfolgt vorzugsweise in Mikroorganismen, wie E. coli. Eine Expression in eukaryontischen Zellen, wie Hefe, CHO-Zellen oder Insektenzellen, ist jedoch auch möglich.

Hierzu werden biologisch funktionelle Plasmide oder virale DNA-Vektoren verwendet, die im wesentlichen die Nukleotide 9 bis 527 von SEQ ID NO:1 enthalten, oder eine Nukleinsäure, die im Rahmen der Degeneration des genetischen Codes für das gleiche Polypeptid codiert, enthalten. Mit derartigen Vektoren werden prokaryontische oder eukaryontische Wirtszellen stabil transformiert oder transfiziert.

Die Expressionsvektoren müssen einen Promotor enthalten, der die Expression des Inhibitorproteins im Wirtsorganismus erlaubt. Derartige Promotoren sind dem Fachmann bekannt und sind beispielsweise lac Promotor (Chang et al., Nature 198 (1977) 1056), trp (Goeddel et al., Nuc. Acids Res. 8 (1980) 4057), $^{\lambda}$PL Promotor (Shimatake et al., Nature 292 (1981) 128) und T5 Promotor (US-Patent Nr. 4,689,406). Ebenfalls geeignet sind synthetische Promotoren wie beispielsweise tac Promotor (US-Patent Nr. 4,551,433). Ebenso geeignet sind gekoppelte Promotorsysteme wie beispielsweise T7-RNA-Polymerase/Promotorsystem (Studier et al., J. Mol. Biol. 189 (1986) 113). Ebenso geeignet sind hybride Promotoren aus einem Bacteriophagen-Promotor und der Operator-Region des Mikroorganismus (EP-A 0 267 851). Zusätzlich zum Promotor ist eine effektive Ribosomenbindungsstelle erforderlich. Für E. coli wird diese Ribosomenbindungsstelle als Shine-Dalgarno (SD)-Sequenz bezeichnet (Shine et al., Nature (1975) 25434; J. Sambrook et al., "Expression of cloned genes in E. coli" in Molecular Cloning: A laboratory manual (1989) Cold Spring Harbor Laboratory Press, USA).

Zur Verbesserung der Expression ist es möglich, das Inhibitorprotein als Fusionsprotein zu exprimieren. In diesem Fall wird üblicherweise eine DNA, welche für den N-terminalen Teil eines endogenen bakteriellen Proteins oder ein anderes stabiles Protein codiert, an das 5'-Ende der für das Inhibitorprotein codierenden Sequenz fusioniert. Beispiele hierfür sind lacZ, trpE.

Nach Expression werden die Fusionsproteine vorzugsweise mit Enzymen (z.B. Faktor Xa) gespalten (Nagai et al., Nature 309 (1984) 810). Weitere Beispiele für die Spaltstellen sind die IgA-Protease-Spaltstelle (WO 91/11520) und die Ubiquitin-Spaltstelle (Miller et al., Bio/Technology 7 (1989) 698).

Das zunächst als inaktive Inclusion Bodies erhaltene rekombinante Protein kann nach dem Fachmann geläufigen Verfahren in lösliches aktives Protein überführt werden. Dazu werden die Inclusion Bodies beispielsweise mit Guanidinhydrochlorid oder Harnstoff in Gegenwart eines Reduktionsmittels solubilisiert, reduziert, das Reduktionsmittel z. B. durch Dialyse entfernt und vorzugsweise unter Verwendung eines Redoxsystems, wie reduziertes und oxidiertes Glutathion renaturiert.

Derartige Methoden sind beispielsweise in US-P 4,933,434, EP-B 0 241 022 und EP-A 0 219 874 beschrieben.

Es ist ebenso möglich, die Proteine als aktive Proteine aus den Mikroorganismen zu sekretieren. Hierzu wird vorzugsweise ein Fusionsprotein verwendet, welches aus der Signalsequenz, die für die Sekretion von Proteinen in den verwendeten Wirtsorganismen geeignet ist (US-Patent Nr. 4,336,336), und der Nukleinsäure, welche für das Inhibitorprotein codiert, besteht. Das Protein wird dabei entweder in das Medium (bei grampositiven Bakterien) oder in den periplasmatischen Raum (bei gramnegativen Bakterien) sekretiert. Zwischen der Signalsequenz und der für den Inhibitor codierenden Sequenz ist zweckmäßig eine Spaltstelle angebracht, die entweder bei der Prozessierung oder in einem zusätzlichen Schritt die Abspaltung des Inhibitorproteins erlaubt. Derartige Signalsequenzen sind beispielsweise ompA (Ghrayeb et al., EMBO J. 3 (1984) 2437), und phoA (Oka et al., Proc. Natl. Acad. Sci. USA 82 (1985) 7212).

Zusätzlich enthalten die Vektoren noch Terminatoren. Terminatoren sind DNA-Sequenzen, die das Ende eines Transkriptionsvorganges signalisieren. Sie zeichnen sich meist durch zwei strukturelle Eigenarten aus: eine umgekehrt repetitive G/C-reiche Region, die intramolekular eine Doppelhelix bilden kann, sowie eine Anzahl von U(bzw. T)-Resten. Beispiele sind trp-Attenuator und Terminator in der DNA des Phagen fd sowie rrnB (Brosius et al., J. Mol. Biol.

148 (1981) 107- 127).

Zusätzlich enthalten die Expressionsvektoren üblicherweise einen selektierbaren Marker, um transformierte Zellen zu selektieren. Derartige selektierbare Marker sind beispielsweise die Resistenzgene für Ampicillin, Chloramphenicol, Erythromycin, Kanamycin, Neomycin und Tetracyclin (Davies et al., Ann. Rev. Microbiol. 32 (1978) 469). Ebenso geeignete selektierbare Marker sind die Gene für essentielle Substanzen der Biosynthese von für die Zelle notwendigen Stoffen wie z.B. Histidin, Tryptophan und Leucin.

Es sind eine Vielzahl von geeigneten bakteriellen Vektoren bekannt. Beispielsweise sind für die folgenden Bakterien Vektoren beschrieben: Bacillus subtilis (Palva et al., Proc. Natl. Acad. Sci. USA 79 (1982) 5582), E. coli (Aman et al., Gene 40 (1985) 183; Studier et al., J. Mol. Biol. 189 (1986) 113), Streptococcus cremoris (Powell et al., Appl. Environ. Microbiol. 54 (1988) 655), Streptococcus lividans und Streptomyces lividans (US-Patent Nr. 4,747,056).

Eine Expression des Inhibitorproteins ist außer in prokaryontischen Mikroorganismen auch in Eukaryonten (wie beispielsweise CHO-Zellen, Hefe oder Insektenzellen) möglich. Als eukaryontisches Expressionssystem werden Hefe- und Insektenzellen bevorzugt. Die Expression in Hefe kann über drei Arten von Hefevektoren (integrierende $YI_P$ (yeast integrating plasmids)-Vektoren, replizierende $YR_P$ (yeast replicon plasmids)-Vektoren und episomale $YE_P$ (yeast episomal plasmids)-Vektoren erfolgen. Näheres hierzu ist beispielsweise in S.M. Kingsman et al., Tibtech 5 (1987) 53 - 57 beschrieben.

Weitere gentechnologische Verfahren zur Herstellung und Expression von geeigneten Vektoren sind in J. Sambrook et al., "Expression of cloned genes in E. coli" in Molecular Cloning: A laboratory manual (1989) Cold Spring Harbor Laboratory Press, USA) beschrieben.

Nach Herstellung erfolgt die Reinigung von rekombinantem ETI chromatographisch über einen Anionenaustauscher, z.B. eine Q-Sepharose®-Säule, einen Kationenaustauscher (z.B. auf Sulfopropylbasis) oder über eine Nickelchelatsäule wie beispielsweise in Porath, J. & Olin, B., Biochemistry 22 (1983), 1621 - 1630 beschrieben. Überraschenderweise wird nach diesem Reinigungsverfahren ein rekombinanter ETI erhalten, dessen inhibitorische Aktivität gegenüber Serinproteasen wie Trypsin und Gewebsplasminogenaktivator wesentlich höher ist als die inhibitorische Aktivität von natürlichem ETI.

Ein so hergestelltes und gereinigtes Polypeptid besitzt die Aktivität eines Inhibitors DE-3 aus Erythrina caffra und ist erhältlich durch Kultivieren von prokaryontischen oder eukaryontischen Wirtszellen, die mit einer exogenen Nukleinsäure (vorzugsweise DNA), welche im wesentlichen der Sequenz Nukleotid 9 bis 527 von SEQ ID NO:1 entspricht, oder einer Nukleinsäure, die im Rahmen der Degeneration des genetischen Codes für das gleiche Polypeptid codiert, in einer Weise transformiert oder transfiziert sind, die es den Wirtszellen erlaubt, unter geeigneten Nährstoffbedingungen das Polypeptid zu exprimieren, und Isolierung des gewünschten Polypeptids, welches eine spezifische inhibitorische Aktivität von ca. 1,07 U/mg oder höher (vorzugsweise 1,07 bis 1,8 U/mg) gegen Trypsin besitzt. Bei verschiedenen Chargen von rekombinantem ETI wurde als spezifische Aktivität beispielsweise 1,2, 1,5 und 1,6U/mg gefunden. Diese Aktivität wird nach chromatographischer Reinigung an einem Anionenaustauscher, Kationenaustauscher oder an einer Nickelchelat-Säule erhalten.

Ein weiterer Gegenstand der Erfindung ist ein Verfahren zur Herstellung eines rekombinanten Polypeptids, welches die Aktivität eines Inhibitors DE-3 aus Erythrina caffra (ETI) besitzt, durch Kultivieren von prokaryontischen oder eukaryontischen Wirtszellen, die mit einer exogenen Nukleinsäure (vorzugsweise DNA), welche im wesentlichen der Sequenz Nukleotid 9 - 527 von SEQ ID NO:1 entspricht, oder einer Nukleinsäure, die im Rahmen der Degeneration des genetischen Codes für das gleiche Polypeptid codiert, in einer Weise transformiert oder transfiziert sind, die es den Wirtszellen erlaubt, unter geeigneten Nährstoffbedingungen das Polypeptid zu exprimieren, Isolierung des Polypeptids aus den Wirtszellen und chromatographische Reinigung an einem Aniontauscher, Kationenaustauscher oder an einer Nickelchelatsäule.

Die Reinigung von Serinproteasen unter Verwendung von rekombinantem ETI erfolgt nach den dem Fachmann geläufigen Verfahren (vgl. z.B. F.J. Joubert (1987)). Hierzu wird ETI kovalent an eine Matrix (z.B. CNBr-Sepharose-Säule) gebunden und das Proteingemisch, welches die Serinprotease enthält, bei neutralen oder schwach alkalischen Bedingungen auf die Säule gegeben und eine Chromatographie durchgeführt. Die Elution erfolgt durch pH-Absenkung ≤ pH 5,5 oder durch Verwendung von Pufferlösungen, welche chaotrope Agenzien, wie z.B. KSCN, enthalten. Das Eluat hat eine Proteinreinheit von über 95% bezogen auf die Serinprotease. Zur weiteren Verwendung wird die Serinprotease zweckmäßig in die jeweils gewünschte Pufferlösung durch Dialyse überführt.

Die Immobilisierung des Inhibitors und alle weiteren Verfahrensschritte zur Reinigung von Serinprotease und t-PA können in analoger Weise wie für den aus E. caffra isolierten Inhibitor DE-3 durchgeführt werden. Derartige Verfahren sind beispielsweise beschrieben in der EP-B 0 218 479, EP-B 0 112 122, USP 4,902,623. Die Immobilisierung erfolgt zweckmäßig an einen inerten Träger, vorzugsweise an CNBr-Sepharose®.

Die nachfolgenden Beispiele und die Sequenzprotokolle beschreiben die Erfindung weiter.

**Beispiel 1**

**Expression von ETI in E. coli**

**a) Gensynthese**

Unter Benutzung der von E. coli bevorzugten Codons wurde aus der Aminosäuresequenz des ETI aus Erythrina caffra (Joubert und Dowdle, Thrombosis and Haemostasis 57 (3) (1987) 356 - 360) eine entsprechende Nukleinsäuresequenz abgeleitet und nach der Methode von Beattie und Fowler (Nature 352 (1991) 548 - 549) synthetisch dargestellt. Um die Klonierung zu erleichtern, wurde an das 5'-Ende eine Schnittstelle für das Restriktionsenzym EcoRI und an das 3'-Ende eine Schnittstelle für das Restriktionsenzym HindIII eingefügt. Die synthetisierte Nukleinsäure wurde mit den Enzymen EcoRI und HindIII restringiert und mit dem Klonierungsvektor pBS+ (Stratagene, US, Catalogue No. 211201, Derivat des Phagen f1 und Stratagene's pBS Plasmid mit $T_3$ und $T_7$ Promotor-Gen, Ampicillin-Resistenz-Gen, f1 origin, ColE-1 origin, lacl Gen, lacZ Gen und einer Multiple Cloning Site), der zuvor ebenfalls mit EcoRI und HindIII verdaut wurde, ligiert. Der Ligationsansatz wurde in Escherichia coli transformiert. Die erhaltenen Klone, selektiert auf Ampicillin, wurden durch Restriktion mit den Enzymen EcoRI und HindIII analysiert. Der resultierende Klon, pBS+ETI, enthält ein zusätzliches EcoRI/HindIII-Fragment mit einer Größe von 539 bp mit SEQ ID NO:1.

**b) Expressionsvektor**

Das Plasmid pBS+ETI wurde mit den Restriktionsenzymen EcoRI und HindIII restringiert, und das 539 bp große Fragment wurde isoliert. Der Expressionsvektor pBTacl (von Boehringer Mannheim GmbH, Katalog Nr. 1081365, auf Basis von pUC8, H. Haymerle et al., Nucl. Acid Res. 14 (1986) 8615 - 8624) wurde ebenfalls mit den Enzymen EcoRI und HindIII verdaut und das etwa 4.6 kb große Vektorfragment isoliert. Beide Fragmente wurden ligiert und zusammen mit dem Helferplasmid pUBS520 (Brinkmann et al., Gene 85 (1989) 109 - 114), das das lac-Repressorgen enthält, in E. coli (DSM 5443) transformiert. Die Selektion der Klone erfolgte über die durch die Plasmide vermittelte Ampicillin- bzw. Kanamycin-Resistenz. Das erhaltene Plasmid pBTETI enthält, im Vergleich zum Ausgangsvektor pBTacl, ein zusätzliches EcoRI/HindIII-Fragment mit einer Größe von 539 bp.

In analoger Weise kann anstelle von DSM 5443 auch DSM 3689, der bereits ein Iq Plasmid enthält, verwendet werden. In diesem Fall ist das Helferplasmid pUB520 nicht erforderlich.

**c) Expression von rekombinantem ETI (recETI) in E. coli**

Zur Überprüfung der Expressionsleistung wurde der E. coli Stamm DSM 5443 mit den Plasmiden pBTETI und pUBS520 in LB-Medium (Sambrook et al., Molecular Cloning (1989) Cold Spring Harbor) in Gegenwart von Ampicillin und Kanamycin (jeweils 50 µg/ml Endkonzentration) bis zu einer optischen Dichte (OD) von 0,6 bei 550 nm angezüchtet. Die Expression wurde durch Zugabe von 5 mM IPTG initiiert. Die Kultur wurde für weitere 4 Stunden inkubiert. Im Anschluß daran wurden die E. coli durch Zentrifugation gesammelt und in Puffer resuspendiert (50 mM Tris-HCl pH 8, 50 mM EDTA); durch Beschallung wurde die Lyse der E. coli herbeigeführt. Durch erneute Zentrifugation wurden die unlöslichen Proteinfraktionen (Inclusion Bodies) gesammelt und in oben genanntem Puffer durch Beschallung resuspendiert. Die Suspension wurde mit 1/4 Volumen Auftragspuffer (250 mM Tris-HCl pH 6.8, 0.01 M EDTA, 5% SDS, 5% Mercaptoethanol, 50% Glycerin und 0.005% Bromphenolblau) versetzt und mit Hilfe eines 12.5% SDS-Polyacrylamidgel analysiert. Als Kontrolle wurde die gleiche Präparation mit einer Kultur von E. coli (pBTETI/pUBS520), die nicht mit IPTG versetzt worden war, durchgeführt und im Polyacrylamidgel aufgetragen. In der Präparation der IPTG-induzierten Kultur kann man, nach Anfärben des Gels mit 0.2% Coomassie-Blue R250 (gelöst in 30% Methanol und 10% Essigsäure) und Entfärben des Gels in einem Methanol-Essigsäure-Gemisch, eine deutliche Bande mit einem Molekulargewicht von etwa 22 kD erkennen. Diese Bande ist in der Präparation der nicht-induzierten E. coli Zellen nicht vorzufinden.

**Beispiel 2**

**Renaturierung und Reinigung von recETI**

50 g Inclusion Bodies (IBs) wurden mit 0,1 M Tris/HCl, pH 8,5, 6 M Guanidin, 0,1 M DTE, 1 mM EDTA solubilisiert (90 min bei 25°C, $C_{Prot.}$ = 10 mg/ml) und nach Einstellen des pH-Wertes auf 2,5 (HCl) gegen 3 mol/l Guanidin/HCl dialysiert. Das Dialysat wurde zentrifugiert (SS34, 13.000 Upm) und durch Konzentrieren über YM 10 auf $C_{Prot.}$ = 36,9 mg/ml eingestellt. Ein 1 l Reaktionsgefäß wurde mit 0,1 M Tris/HCl, pH 8,5, l mM EDTA, l mM GSH, 0,1 mM GSSG gefüllt. Die Renaturierung wurde bei 20°C durch 16-fache Zugabe des Dialysats (jeweils 600 µg Protein/ml Puffer) im Zeitabstand von 30 min durchgeführt.
Die Renaturierung ergibt 2,8 U/ml aktives ETI.

**Reinigung von recETI**

**a) über Anionenaustauscher**

recETI wird in 0,1M Tris/HCl, pH 8,5, 1 mM EDTA, 1 mM GSH, 0,1mM GSSG renaturiert. Das Renaturat wird mit $H_2O$ 1:2 verdünnt, mit HCl auf pH 8,0 eingestellt, gegen 50 mM Tris/HCl pH 8,0 dialysiert und auf eine mit 50 mM Tris/HCl, pH 8,0 äquilibrierte Q-Sepharose®-Säule aufgetragen (5 mg Protein/ml Gel). Nach Waschen der Säule mit dem Äquilibrierungspuffer und mit 50 mM $Na_2HPO_4/H_3PO_4$, pH 8,0 (jeweils fünf Säulenvolumen) erfolgt die Elution mit 50 mM $Na_2HPO_4/H_3PO_4$, pH 8,0, 0,2 M NaCl.

**b) über Kationenaustauscher**

Renaturiertes ETI wurde durch Zugabe von HCl auf pH 4,0 eingestellt und gegen 50 mM NaOAc/HCl, pH 4,0 dialysiert (Cross Flow). Das Dialysat wurde zentrifugiert (13.000 Upm, 30 min, SS 34) und auf eine mit 50 mM NaOAc/HCl, pH 4,0 äquilibrierte TSK-SP-Säule (Kationenaustauscher mit Sulfopropylseitengruppen, Merck, Deutschland, Volumen 15 ml) aufgetragen. Nach Waschen der Säule mit dem Äquilibrierungspuffer und mit 50 mM NaOAc/HCl, pH 4,0, 0,1 M NaCl erfolgt die Elution 50 mM NaOAc/HCl, pH 4,0, 0,2 M NaCl.
Die Reinheit des Eluats wurde durch SDS-PAGE und über RP-HPLC überprüft.

Ergebnis:

ETI bindet unter den verwendeten Bedingungen an die TSK-SP-Säule und kann mit 0,2 M NaCl eluiert werden. SDS-PAGE- und RP-HPLC-Analytik ergeben eine Reinheit von > 95%.

**Beispiel 3**

**Vergleich der spezifischen Aktivität von recETI und von ETI aus dem Samen von Erythrina caffra**

recETI und ETI, isoliert aus dem Samen von E. caffra, wurden gegen 50 mM $Na_2HPO_4/H_3PO_4$, pH 8,0, 0.2 M NaCl dialysiert und auf eine Proteinkonzentration von 0.8 mg/ml eingestellt. Die Proteinkonzentration wurde durch Messung der UV-Absorption bei 280 nm bestimmt ($\varepsilon$ = 1,46 $cm^2$/mg).

**Bestimmung der ETI-Aktivität**

Die Hemmung von Trypsin durch ETI wird mit N-$\alpha$-Benzoylethylester (BAEE) als Substrat gemessen. 40 µl einer Trypsin-Lösung (0,13 mg/ml 2 mM HCl) werden in einer Quarzküvette mit 60 µl Testpuffer (0,1 M Tris/HCl, pH 8,0) und 100 µl ETI-Lösung gemischt und 5 min bei 30°C inkubiert. Nach Zugabe von 800 µl BAEE-Lösung (20 mg BAEE x HCl/100 ml Testpuffer) wird der Exthinktionsanstieg/min bei 253 nm bestimmt.
Die ETI-Aktivität wird nach folgender Formel ermittelt:

$$U/ml = [1 - E_{Probe}/E_{Trypsin}] \cdot C_{Trypsin} \cdot 0.328 \cdot V$$

$E_{Probe}$:  Extinktionsanstieg/min der gehemmten Probe
$E_{Trypsin}$:  Extinktionsanstieg/min des ungehemmten Trypsins
$C_{Trypsin}$:  Trypsin-Konzentration im Testansatz
V:  Vorverdünnung der ETI-Lösung

| Protein | $C_{Prot.}$ (mg/ml) | Aktivität (U/ml) | Spez. Aktivität U/mg) |
|---|---|---|---|
| ETI (Samen) | 0.81 | 0.71 | 0.88 |
| recETI | 0.83 | 0.89 | 1.07 |

Ergebnis: Die spezifische Aktivität von recETI ist um 20% höher als die spezifische Aktivität des nach klassischen Verfahren aus dem Samen von E. caffra isolierten ETI.

Bei weiteren Chargen von rekombinantem ETI wurde als spezifische Aktivität beispielsweise 1,2, 1,5 und 1,6 U/mg gefunden.

**Beispiel 4**

**Kopplung von recETI an CNBr-Sepharose®**

170 mg gereinigtes recETI wurden gegen 0,05 M $H_3BO_3$/NaOH, pH 8,0, 0,5 M NaCl (Kupplungspuffer) dialysiert und mit 7,5 g CNBr-Sepharose® (über Nacht in 500 ml 1 mM HCl gequollen, danach abgesaugt und in Kupplungspuffer suspendiert) gemischt. Die Suspension wurde 90 min bei Raumtemperatur inkubiert, abgesaugt und über Nacht mit 400 ml 0,1 M Tris/HCl, pH 8,0 geschüttelt. Die recETI-Sepharose® wurde abgesaugt und mit 0,7 M Arginin/$H_3PO_4$, pH 7,5 äquilibriert.

**Beispiel 5**

**Reinigung eines rekombinanten Plasminogenaktivators**

54 mg rekombinanter Plasminogenaktivator K2P (hergestellt nach WO 90/09437 oder USP 5,223,256) wurden auf eine mit 0,7 M Arginin/$H_3PO_4$, pH 7,5 äquilibrierte recETI-Sepharose gegeben. Nach Waschen mit dem Äquilibrierungspuffer und mit 0,3 M Arginin/$H_3PO_4$, pH 7,0 (jeweils fünf Säulenvolumen) erfolgte die Elution mit 0,3 M Arginin/$H_3PO_4$, pH 4,5. Der Plasminogenaktivator-Gehalt im Eluat wurde mit S 2288 als Substrat bestimmt (Kohnert et al., Prot. Engineer. 5 (1992) 93 - 100).

Ergebnis: Die Bindungskapazität der recETI-Sepharose für den Plasminogenaktivator beträgt 1,2 mg (entsprechend 0,63 MU) Plasminogenaktivator/ml recETI-Sepharose.

**Patentansprüche**

1. Verfahren zur Reinigung von Serinproteasen aus einem Proteingemisch durch Bindung der Serinprotease an ein immobilisiertes Polypeptid mit der Aktivität eines Inhibitors DE-3 aus Erythrina caffra, Entfernen der ungebundenen Anteile aus dem Proteingemisch, Ablösen der Serinprotease vom Inhibitor, Trennung des immobilisierten Inhibitors von der löslichen Serinprotease und Isolierung der Serinprotease, dadurch gekennzeichnet, daß als Polypeptid ein Polypeptid verwendet wird, das das Produkt einer prokaryontischen oder eukaryontischen Expression einer exogenen Nukleinsäure ist und chromatographisch über einen Anionenaustauscher, Kationenaustauscher oder über eine Nickelchelatsäule gereinigt ist.

2. Verfahren nach Anspruch 1, dadurch gekennzeichnet, daß die Serinprotease ein Plasminogenaktivator ist.

3. Verfahren nach Anspruch 2, dadurch gekennzeichnet, daß der Plasminogenaktivator ein Gewebsplasminogenaktivator oder ein Derivat davon ist.

4. Verfahren nach Anspruch 1 bis 3, dadurch gekennzeichnet, daß der Inhibitor an einem inerten Träger immobilisiert ist.

5. Verfahren nach den Ansprüchen 1 bis 4, dadurch gekennzeichnet, daß die exogene Nukleinsäure im wesentlichen der Sequenz Nukleotid 9 bis 527 aus SEQ ID NO:1 oder einer Nukleinsäure, die im Rahmen der Degeneration des genetischen Codes für das gleiche Polypeptid codiert, entspricht.

**6.** Verwendung eines Polypeptids mit der Aktivität eines Inhibitors DE-3 aus Erythrina caffra, das das Produkt einer prokaryontischen oder eukaryontischen Expression einer exogenen Nukleinsäure ist und chromatographisch über einen Anionenaustauscher, Kationenaustauscher oder über Nickelchelat gereinigt ist, zur affinitätschromatographischen Reinigung von Serinproteasen.

**7.** Verwendung nach Anspruch 6, dadurch gekennzeichnet, daß die Serinprotease ein Plasminogenaktivator ist.

**8.** Verwendung nach Anspruch 7, dadurch gekennzeichnet, daß der Plasminogenaktivator ein Gewebsplasminogenaktivator oder ein Derivat davon ist.

**9.** Verfahren zur Herstellung eines Polypeptids, welches die Aktivität eines Inhibitors DE-3 aus Erythrina caffra besitzt, durch Kultivieren von prokaryontischen oder eukaryontischen Wirtszellen, die mit einer exogenen Nukleinsäure, welche im wesentlichen der Sequenz Nukleotid 9 bis 527 aus SEQ ID NO:1 oder einer Nukleinsäure, die im Rahmen der Degeneration des genetischen Codes für das gleiche Polypeptid codiert, entspricht, in einer Weise transformiert oder transfiziert sind, die es den Wirtszellen erlauben, unter geeigneten Nährstoffbedingungen das Polypeptid zu exprimieren, und Isolierung des gewünschten Polypeptids, welches dadurch gekennzeichnet ist, daß es eine spezifische inhibitorische Aktivität von ca. 1,07 U/mg gegen Trypsin besitzt.

**10.** Verfahren nach Anspruch 9, dadurch gekennzeichnet, daß als Nukleinsäure eine Nukleinsäure der Sequenz Nukleotid 9 bis 527 aus SEQ ID NO:1 oder eine Nukleinsäure, die im Rahmen der Degeneration des genetischen Codes für das gleiche Polypeptid codiert, verwendet wird.

**11.** Verfahren nach Anspruch 9 oder 10, dadurch gekennzeichnet, daß die Wirtszellen E. coli-Zellen sind.

**12.** Verfahren nach Anspruch 9 oder 10, dadurch gekennzeichnet, daß die Wirtszellen Hefezellen oder CHO-Zellen sind.

**13.** Nukleinsäure der Nukleotide 9 bis 527 aus SEQ ID NO:1, die für ein Polypeptid mit der Aktivität eines Inhibitors DE-3 aus Erythrina caffra codiert.

**14.** Biologisch funktionelles Plasmid oder viraler DNA-Vektor, der eine Nukleinsäure nach Anspruch 13 enthält.

**15.** Prokaryontische oder eukaryontische Wirtszellen, welche mit einem DNA-Vektor nach Anspruch 14 stabil transformiert oder transfiziert sind.

**16.** Polypeptid, welches die Aktivität eines Inhibitors DE-3 aus Erythrina caffra besitzt und erhältlich ist durch Kultivieren von prokaryontischen oder eukaryontischen Wirtszellen, die mit einer exogenen Nukleinsäure, welche im wesentlichen der Sequenz Nukleotid 9 bis 527 aus SEQ ID NO:1 oder einer Nukleinsäure, die im Rahmen der Degeneration des genetischen Codes für das gleiche Polypeptid codiert, entspricht, in einer Weise transformiert oder transfiziert sind, die es den Wirtszellen erlaubt, unter geeigneten Nährstoffbedingungen das Polypeptid zu exprimieren, und Isolierung des gewünschten Polypeptids, welches dadurch gekennzeichnet ist, daß es eine spezifische inhibitorische Aktivität von ca. 1,07 U/mg oder höher gegen Trypsin besitzt.

**17.** Polypeptid nach Anspruch 16, dadurch gekennzeichnet, daß die Expression in prokaryontischen Wirtszellen erfolgt und das Polypeptid die Aminosäuresequenz SEQ ID NO:2 besitzt.

**18.** Polypeptid nach Anspruch 16, dadurch gekennzeichnet, daß die Expression in eukaryontischen Wirtszellen erfolgt und das Polypeptid die Aminosäuresequenz SEQ ID NO:2 ohne N-terminales Methionin besitzt.

**19.** Verfahren zur Herstellung eines rekombinanten Polypeptids, welches die Aktivität eines Inhibitors DE-3 aus Erythrina caffra besitzt, durch Kultivieren von prokaryontischen oder eukaryontischen Wirtszellen, die mit einer exogenen Nukleinsäure, welche im wesentlichen der Sequenz Nukleotid 9 bis 527 von SEQ ID NO:1 entspricht, oder einer Nukleinsäure, die im Rahmen der Degeneration des genetischen Codes für das gleiche Polypeptid codiert, in einer Weise transformiert oder transfiziert sind, die es den Wirtszellen erlaubt, unter geeigneten Nährstoffbedingungen das Polypeptid zu exprimieren, Isolierung des Polypeptids aus den Wirtszellen und chromatographische Reinigung an einem Anionaustauscher, Kationenaustauscher oder an einer Nickelchelatsäule.

## Claims

1. Process for purifying serine proteases from a protein mixture by binding the serine protease to an immobilized polypeptide having the activity of a DE-3 inhibitor from Erythrina caffra, removing unbound components from the protein mixture, detaching the serine protease from the inhibitor, separating the immobilized inhibitor from the soluble serine protease and isolating the serine protease, wherein a polypeptide is used as the polypeptide which is the product of a prokaryotic or eukaryotic expression of an exogenous nucleic acid and is purified chromatographically by means of an anion exchanger, cation exchanger or by means of a nickel chelate.

2. Process as claimed in claim 1, wherein the serine protease is a plasminogen activator.

3. Process as claimed in claim 2, wherein the plasminogen activator is a tissue plasminogen activator or a derivative thereof.

4. Process as claimed in claims 1 to 3, wherein the inhibitor is immobilized on an inert carrier.

5. Process as claimed in claims 1 to 4, wherein the exogenous nucleic acid essentially corresponds to the sequence nucleotides 9 to 527 of SEQ ID NO: 1 or to a nucleic acid which codes for the same polypeptide within the scope of the degeneracy of the genetic code.

6. Use of a polypeptide with the activity of a DE-3 inhibitor from Erythrina caffra, which is the product of a prokaryotic or eukaryotic expression of an exogenous nucleic acid and which is chromatographically purified by means of an anion exchanger, cation exchanger or by means of a nickel chelate, for the affinity-chromatographic purification of serine proteases.

7. Use as claimed in claim 6, wherein the serine protease is a plasminogen activator.

8. Use as claimed in claim 7, wherein the plasminogen activator is a tissue plasminogen activator or a derivative thereof.

9. Process for the production of a polypeptide which has the activity of an inhibitor DE-3 from Erythrina caffra by culturing prokaryotic or eukaryotic host cells which are transformed or transfected with an exogenous nucleic acid which essentially corresponds to the sequence nucleotides 9 to 527 from SEQ ID NO:1 or to a nucleic acid that codes for the same polypeptide within the scope of the degeneracy of the genetic code in a manner that allows the host cells to express the polypeptide under suitable nutrient conditions and isolating the desired polypeptide, wherein it has a specific inhibitory activity towards trypsin of about 1.07 U/mg.

10. Process as claimed in claim 9, wherein a nucleic acid of the sequence nucleotides 9 to 527 from SEQ ID NO: 1 or a nucleic acid that codes for the same polypeptide within the scope of the degeneracy of the genetic code is used as the nucleic acid.

11. Process as claimed in claim 9 or 10, wherein the host cells are E. coli cells.

12. Process as claimed in claim 9 or 10, wherein the host cells are yeast cells or CHO cells.

13. Nucleic acid of nucleotides 9 to 527 from SEQ ID NO: 1 which codes for a polypeptide with the activity of an inhibitor DE-3 from Erythrina caffra.

14. Biologically functional plasmid or viral DNA vector which contains a nucleic acid as claimed in claim 13.

15. Prokaryotic or eukaryotic host cells which are stably transformed or transfected with a DNA vector as claimed in claim 14.

16. Polypeptide which has the activity of an inhibitor DE-3 from Erythrina caffra and which is obtainable by culturing prokaryotic or eukaryotic host cells which are transformed or transfected with an exogenous nucleic acid which essentially corresponds to the sequence nucleotides 9 to 527 from SEQ ID NO:1 or to a sequence that codes for the same polypeptide within the scope of the degeneracy of the genetic code in a manner that allows the host cells to express the desired polypeptide under suitable nutrient conditions and isolating the desired polypeptide, wherein it has a specific inhibitory activity towards trypsin of ca. 1.07 U/mg or more.

17. Polypeptide as claimed in claim 16, wherein the expression is carried out in prokaryotic host cells and the polypeptide has the amino acid sequence SEQ ID NO: 2.

18. Polypeptide as claimed in claim 16, wherein the expression is carried out in eukaryotic host cells and the polypeptide has the amino acid sequence SEQ ID NO: 2 without an N-terminal methionine.

19. Process for the production of a recombinant polypeptide which has the activity of a DE-3 inhibitor from Erythrina caffra by culturing prokaryotic or eukaryotic host cells which are transformed or transfected with an nucleic acid which essentially corresponds to the sequence nucleotides 9 to 527 from SEQ ID NO:1 or to a nucleic acid that codes for the same polypeptide within the scope of the degeneracy of the genetic code in a manner that enables the host cells to express the polypeptide under suitable nutrient conditions and isolating the polypeptide from the host cells and chromatographic purification on an anion exchanger, cation exchanger or on a nickel chelate column.

## Revendications

1. Procédé de purification de sérine-protéases à partir d'un mélange de protéines, par liaison de la sérine-protéase à un polypeptide immobilisé ayant l'activité d'un inhibiteur DE-3 d'Erythrina caffra, élimination de la partie non liée du mélange de protéines, détachement de la sérine-protéase de l'inhibiteur par dissolution, séparation de l'inhibiteur immobilisé de la sérine-protéase soluble et isolement de la serine-protéase, caractérisé par le fait que comme polypeptide, on utilise un polypeptide qui est le produit d'une expression procariote ou eucariote d'un acide nucléique exogène et qui est purifié par chromatographie sur un échangeur d'anions, un échangeur de cations ou une colonne de chélate de nickel.

2. Procédé selon la revendication 1, caractérisé par le fait que la sérine-protéase est un activateur du plasminogène.

3. Procédé selon la revendication 2, caractérisé par le fait que l'activateur du plasminogène est un activateur tissulaire du plasminogène ou un dérivé de celui-ci.

4. Procédé selon l'une quelconque des revendications 1 à 3, caractérisé par le fait que l'inhibiteur est immobilisé sur un support inerte.

5. Procédé selon l'une quelconque des revendications 1 à 4, caractérisé par le fait que l'acide nucléique exogène correspond essentiellement aux nucléotides 9 à 527 de la SEQ ID NO:1 ou à un acide nucléique qui, compte tenu de la dégénérescence du code génétique, code pour le même polypeptide.

6. Utilisation d'un polypeptide ayant l'activité d'un inhibiteur DE-3 d'Erythrina caffra, qui est le produit d'une expression procariote ou eucariote d'un acide nucléique exogène et qui est purifié par chromatographie sur un échangeur d'anions, un échangeur de cations ou une colonne de chélate de nickel, pour la purification par chromatographie d'affinité de sérine-protéases.

7. Utilisation selon la revendication 6, caractérisée par le fait que la sérine-protéase est un activateur du plasminogène.

8. Utilisation selon la revendication 7, caractérisée par le fait que l'activateur du plasminogène est un activateur tissulaire du plasminogène ou un dérivé de celui-ci.

9. Procédé de préparation d'un polypeptide qui présente l'activité d'un inhibiteur DE-3 d'Erythrina caffra, par culture de cellules hôtes procariotes ou eucariotes, qui sont transformées ou transfixées avec un acide nucléique exogène qui correspond essentiellement aux nucléotides 9 à 527 de la SEQ ID NO:1 ou a un acide nucléique qui, compte tenu de la dégénérescence du code génétique, code pour le même polypeptide, de manière à permettre aux cellules hôte d'exprimer le polypeptide dans des conditions de milieu de culture appropriées, et isolement du polypeptide voulu, le polypeptide étant caractérisé par le fait qu'il présente une activité inhibitrice spécifique d'environ 1,07 U/mg vis-à-vis de la trypsine.

10. Procédé selon la revendication 9, caractérisé par le fait que comme acide nucléique, on utilise un acide nucléique correspondant aux nucléotides 9 à 527 de la SEQ ID NO:1 ou un acide nucléique qui, compte tenu de la dégénérescence du code génétique, code pour le même polypeptide.

11. Procédé selon la revendication 9 ou 10, caractérisé par le fait que les cellules hôtes sont des cellules d'E. coli.

12. Procédé selon la revendication 9 ou 10, caractérisé par le fait que les cellules hôtes sont des cellules de levure ou des cellules de CHO.

13. Acide nucléique correspondant aux nucléotides 9 à 527 de la SEQ ID NO:1, qui code pour un polypeptide présentant l'activité d'un inhibiteur DE-3 d'Erythrina caffra.

14. Plasmide biologique fonctionnel ou vecteur d'ADN viral, qui contient un acide nucléique selon la revendication 13.

15. Cellules hôtes procariotes ou eucariotes, qui sont transformées ou transfixées d'une manière stable avec un vecteur d'ADN selon la revendication 14.

16. Polypeptide, qui présente l'activité d'un inhibiteur DE-3 d'Erythrina caffra et qui peut être obtenu par culture de cellules hôtes procariotes ou eucariotes, qui sont transformées ou transfixées avec un acide nucléique exogène qui correspond essentiellement aux nucléotides 9 à 527 de la SEQ ID NO:1 ou un acide nucléique qui, compte tenu de la dégénérescence du code génétique, code pour le même polypeptide, de manière à permettre aux cellules hôtes d'exprimer le polypeptide dans des conditions de milieu de culture appropriées, et isolement du polypeptide voulu, qui est caractérisé par le fait qu'il présente une activité inhibitrice spécifique d'environ 1,07 U/mg ou supérieure vis-à-vis de la trypsine.

17. Polypeptide selon la revendication 16, caractérisé par le fait que l'expression est réalisée des dans cellules hôtes procariotes et que le polypeptide présente la séquence d'acides aminés SEQ ID NO:2.

18. Polypeptide selon la revendication 16, caractérisé par le fait que l'expression est réalisée dans des cellules hôtes eucariotes et que le polypeptide présente la séquence d'acides aminés SEQ ID NO:2, sans méthionine N-terminale.

19. Procédé de préparation d'un polypeptide recombinant, qui présente l'activité d'un inhibiteur DE-3 d'Erythrina caffra et qui peut être obtenu par culture de cellules hôtes procariotes ou eucariotes, qui sont transformées ou transfixées avec un acide nucléique exogène qui correspond essentiellement aux nucléotides 9 à 527 de la SEQ ID NO: 1 ou un acide nucléique qui, compte tenu de la dégénérescence du code génétique, code pour le même polypeptide, de manière à permettre aux cellules hôtes d'exprimer le polypeptide dans des conditions de milieu de culture appropriées, isolement du polypeptide voulu à partir des cellules hôtes et purification chromatographique sur un échangeur d'anions, un échangeur de cations ou une colonne de chélate de nickel.

SEQUENZPROTOKOLL

(1) ALLGEMEINE INFORMATION:

    (i) ANMELDER:

        (A) NAME: BOEHRINGER MANNHEIM GMBH
        (B) STRASSE: Sandhofer Str. 116
        (C) ORT: Mannheim
        (E) LAND: Germany
        (F) POSTLEITZAHL: D-68305
        (G) TELEPHON: 0621/759-3197
        (H) TELEFAX: 0621/759-4457

    (ii) ANMELDETITEL: Verwendung von rekombinantem Inhibitor aus Erythrina caffra zur Reinigung von Serinproteasen

    (iii) ANZAHL DER SEQUENZEN: 2

    (iv) COMPUTER-LESBARE FORM:

        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.25 (EPA)

(2) INFORMATION ZU SEQ ID NO: 1:

    (i) SEQUENZ CHARAKTERISTIKA:

        (A) LÄNGE: 539 Basenpaare
        (B) ART: Nukleinsäure

(C) STRANGFORM: Doppel

(D) TOPOLOGIE: linear

(ii) ART DES MOLEKÜLS: cDNS

(ix) MERKMALE:

(A) NAME/SCHLÜSSEL: CDS

(B) LAGE: 9..527

(D) SONSTIGE ANGABEN: /note= "Met only included in prokaryontic expression"

(ix) MERKMALE:

(A) NAME/SCHLÜSSEL: misc_feature

(B) LAGE: 1..8

(D) SONSTIGE ANGABEN: /function= "multiple cloning site"

(ix) MERKMALE:

(A) NAME/SCHLÜSSEL: misc_feature

(B) LAGE: 528..539

(D) SONSTIGE ANGABEN: /function= "multiple cloning site"

(xi) SEQUENZBESCHREIBUNG: SEQ ID NO: 1:

```
GAATTCTT ATG GTA TTA TTA GAT GGT AAC GGC GAA GTG GTG CAG AAC GGC   50
         Met Val Leu Leu Asp Gly Asn Gly Glu Val Val Gln Asn Gly
          1               5                  10

GGT ACC TAT TAT CTG CTG CCG CAG GTG TGG GCG CAG GGC GGC GGC GTG   98
Gly Thr Tyr Tyr Leu Leu Pro Gln Val Trp Ala Gln Gly Gly Gly Val
 15              20                  25                      30

CAG CTG GCG AAA ACC GGC GAA GAA ACC TGC CCG CTG ACC GTG GTG CAG  146
Gln Leu Ala Lys Thr Gly Glu Glu Thr Cys Pro Leu Thr Val Val Gln
             35                  40                  45

AGC CCG AAC GAA CTG AGC GAT GGC AAA CCG ATT CGT ATT GAA AGC CGT  194
Ser Pro Asn Glu Leu Ser Asp Gly Lys Pro Ile Arg Ile Glu Ser Arg
             50                  55                  60

CTG CGT AGC GCG TTT ATT CCG GAT GAT GAT AAA GTG CGT ATT GGC TTT  242
Leu Arg Ser Ala Phe Ile Pro Asp Asp Asp Lys Val Arg Ile Gly Phe
             65                  70                  75

GCG TAT GCG CCG AAA TGC GCG CCG AGC CCG TGG TGG ACC GTG GTG GAA  290
Ala Tyr Ala Pro Lys Cys Ala Pro Ser Pro Trp Trp Thr Val Val Glu
         80                  85                  90

GAT GAA CAG GAA GGC CTG AGC GTG AAA CTG AGC GAA GAT GAA AGC ACC  338
Asp Glu Gln Glu Gly Leu Ser Val Lys Leu Ser Glu Asp Glu Ser Thr
 95                  100                 105                 110

CAG TTT GAT TAT CCG TTT AAA TTT GAA CAG GTG AGC GAT CAG CTG CAT  386
Gln Phe Asp Tyr Pro Phe Lys Phe Glu Gln Val Ser Asp Gln Leu His
                 115                 120                 125

AGC TAT AAA CTG CTG TAT TGC GAA GGC AAA CAT GAA AAA TGC GCG AGC  434
Ser Tyr Lys Leu Leu Tyr Cys Glu Gly Lys His Glu Lys Cys Ala Ser
             130                 135                 140

ATT GGC ATT AAC CGT GAT CAG AAA GGC TAT CGT CGT CTG GTG GTG ACC  482
Ile Gly Ile Asn Arg Asp Gln Lys Gly Tyr Arg Arg Leu Val Val Thr
             145                 150                 155

GAA GAT TAT CCG CTG ACC GTG GTG CTG AAA AAA GAT GAA AGC AGC      527
Glu Asp Tyr Pro Leu Thr Val Val Leu Lys Lys Asp Glu Ser Ser
 160             165                 170

TGATAAAAGC TT                                                    539
```

(2) INFORMATION ZU SEQ ID NO: 2:

    (i) SEQUENZ CHARAKTERISTIKA:

        (A) LÄNGE: 173 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 2:

```
Met Val Leu Leu Asp Gly Asn Gly Glu Val Val Gln Asn Gly Gly Thr
 1               5                  10                  15

Tyr Tyr Leu Leu Pro Gln Val Trp Ala Gln Gly Gly Gly Val Gln Leu
             20                  25                  30

Ala Lys Thr Gly Glu Glu Thr Cys Pro Leu Thr Val Val Gln Ser Pro
         35                  40                  45

Asn Glu Leu Ser Asp Gly Lys Pro Ile Arg Ile Glu Ser Arg Leu Arg
     50                  55                  60

Ser Ala Phe Ile Pro Asp Asp Asp Lys Val Arg Ile Gly Phe Ala Tyr
 65                  70                  75                  80

Ala Pro Lys Cys Ala Pro Ser Pro Trp Trp Thr Val Val Glu Asp Glu
             85                  90                  95

Gln Glu Gly Leu Ser Val Lys Leu Ser Glu Asp Glu Ser Thr Gln Phe
            100                 105                 110

Asp Tyr Pro Phe Lys Phe Glu Gln Val Ser Asp Gln Leu His Ser Tyr
        115                 120                 125

Lys Leu Leu Tyr Cys Glu Gly Lys His Glu Lys Cys Ala Ser Ile Gly
    130                 135                 140

Ile Asn Arg Asp Gln Lys Gly Tyr Arg Arg Leu Val Val Thr Glu Asp
145                 150                 155                 160

Tyr Pro Leu Thr Val Val Leu Lys Lys Asp Glu Ser Ser
            165                 170
```